# EUROPÄISCHE PATENTSCHRIFT

(11) **EP 0 827 752 B1**
(45) Veröffentlichungstag und Bekanntmachung des Hinweises auf die Patenterteilung: **19.12.2001**
(21) Anmeldenummer: 96114199.1
(22) Anmeldetag: 04.09.1996
(51) Int. Cl.: A61L 9/03, A01M 1/20

(54) **Elektrische Vorrichtung zum Verdunsten von Wirkstoffen**
Electric vaporizer for active substances
Appareil électrique de vaporisation de substances actives

(43) Veröffentlichungstag der Anmeldung: 11.03.1998
(73) Patentinhaber: Steinel GmbH & Co. KG, 33442 Herzebrock-Clarholz (DE)
(72) Erfinder: Steinel Jr., Heinrich Wolfgang, 86825 Bad Wörishofen (DE)
(74) Vertreter: Grünecker, Kinkeldey, Stockmair & Schwanhäusser Anwaltssozietät

(56) Entgegenhaltungen:
- EP-A- 0 695 553
- EP-A- 0 696 457
- DE-A- 1 665 063

## Beschreibung

Die vorliegende Erfindung bezieht sich auf eine elektrische Vorrichtung zum Verdunsten von Wirkstoffen, Parfümen od. dgl. gemäß dem Oberbegriff des Patentanspruchs 1.

Derartige Vorrichtungen sind durch die EP 686 457 A1 und die EP 695 553 A1 bekannt geworden.

Die in diesen beiden Anmeldungen beschriebene Vorrichtung weist eine zweigeteilte elektrische Schaltung auf, die aus zwei aufeinandergelegten, kunststoffumspritzten Stanzblechteilen besteht. Bei der Herstellung wird ein einstückiges Stanzblechteil mit einem Kunststoffmaterial umspritzt, und vor der Montage in zwei Teile getrennt. Weiterhin müssen überstehende Stanzblechteile abgetrennt werden und es sind Biegevorgänge erforderlich, um eine elektrische Verbindung zwischen den beiden aufeinandergelegten Teilen herstellen zu können. Schließlich muß eine mechanische Verbindung zwischen den Schaltungsteilen hergestellt werden, beispielsweise durch eine aufgeschobene Metallklammer. Diese zusätzlichen Arbeitsschritte bedeuten in der Massenfertigung einen erheblichen Kostenfaktor und insbesondere das Aufeinanderlegen von zwei Teilen erfordert entweder hochkomplizierte Positionierungseinrichtungen oder teuere Handarbeit.

Die Aufgabe der vorliegenden Erfindung ist es daher, eine Vorrichtung der genannten Art so weiterzubilden, daß sie möglichst einfach und kostengünstig herstellbar ist.

Dies wird von einer Vorrichtung mit den Merkmalen des Patentanspruchs 1 gelöst.

Bei der elektrischen Vorrichtung nach der vorliegenden Erfindung sind die aufeinandergestapelten Schaltungsteile mittels eines einstückig ausgebildeten Stanzblechteils mechanisch und elektrisch miteinander gekoppelt. Während der Montage der Vorrichtung werden die Schaltungsteile einfach aufeinandergeklappt, wobei die mechanische Kopplung wie ein Scharnier die Schaltungsteile zueinanderführt. Daher ist es nicht notwendig, wie beim Stand der Technik, zwei voneinander getrennte Schaltungsteile zu ergreifen und zueinander zu positionieren. Durch die mechanische Kopplung entfällt auch die Notwendigkeit einer zusätzlichen Klammer, um die beiden Schaltungsteile in Verbindung zu halten.

Gleichzeitig wird durch das einstückig ausgebildete Stanzblechteil eine elektrische Verbindung zwischen beiden Schaltungsteilen geschaffen, welche beim Stand der Technik zusätzliche Biegevorgänge erforderte.

Die erfindungsgemäße Vorrichtung besteht somit aus weniger Teilen, besitzt einen einfacheren Aufbau und ist wesentlich leichter zusammenbaubar, was erhebliche Kostenvorteile bei der Herstellung mitsichbringt.

Vorzugsweise sind in wenigstens einem Spritzgußteil Ausnehmungen zur Aufnahme von diskreten elektrischen Bauelementen eingeformt, was die Bestückung der Schaltung erleichtert.

Insbesondere ist es von Vorteil, wenn den Ausnehmungen des einen Spritzgußteils korrespondierende hervorstehende Haltevorsprünge auf dem anderen Spritzgußteil zugeordnet sind, da dadurch beim Aufeinanderklappen der beiden Schaltungsteile die Anschlußdrähte der Bauelemente klemmend zwischen den Haltevorsprüngen und dem Stanzblechteil gehalten werden. Somit entfällt beim Bestücken der Schaltung mit elektrischen Bauelementen ein Einlöten oder Punktschweißen, um eine elektrisch leitende Verbindung zwischen den Anschlußdrähten der Bauelemente und dem Stanzblechteil zu schaffen.

Gemäß einer bevorzugten Ausführung sind in wenigstens ein Spritzgußteil Ausnehmungen eingeformt, die mit entsprechenden Haltevorsprüngen auf dem anderen Spritzgußteil in rastenden Eingriff bringbar sind. Dies erleichtert eine genaue Positionierung der beiden Schaltungsteile und stellt eine leicht herstellbare Schnapp-Verbindung bereit.

In einer weiteren bevorzugten Ausgestaltung weist die Vorrichtung elektrische Anschlußsteckerstifte auf, die sich in den Innenraum des Gehäuses erstrecken. Zur formschlüssigen Aufnahme der Anschlußsteckerstifte sind in ein Kunststoffspritzgußteil entsprechende Aufnahmeöffnungen eingeformt. Dies ermöglicht einen direkten Anschluß der elektrischen Schaltung an eine Spannungsversorgung ohne die Verwendung von Litzendrähten, was den Herstellungsaufwand für die Vorrichtung vermindert.

Eine bevorzugte Ausführung der vorliegenden Erfindung wird nachfolgend unter Bezugnahme auf die begleitenden Figuren beschrieben, welche zeigen:
Fig. 1 eine perspektivische Explosivdarstellung der erfindungsgemäßen Vorrichtung,
Fig. 2 eine perspektivische Ansicht der elektrischen Schaltung der erfindungsgemäßen Vorrichtung der Fig. 1 vor der Montage,
Fig. 3 eine perspektivische Ansicht der elektrischen Schaltung der Fig. 2 während der Montage, und
Fig. 4 eine fertig montierte und bestückte elektrische Schaltung der erfindungsgemäßen Vorrichtung.

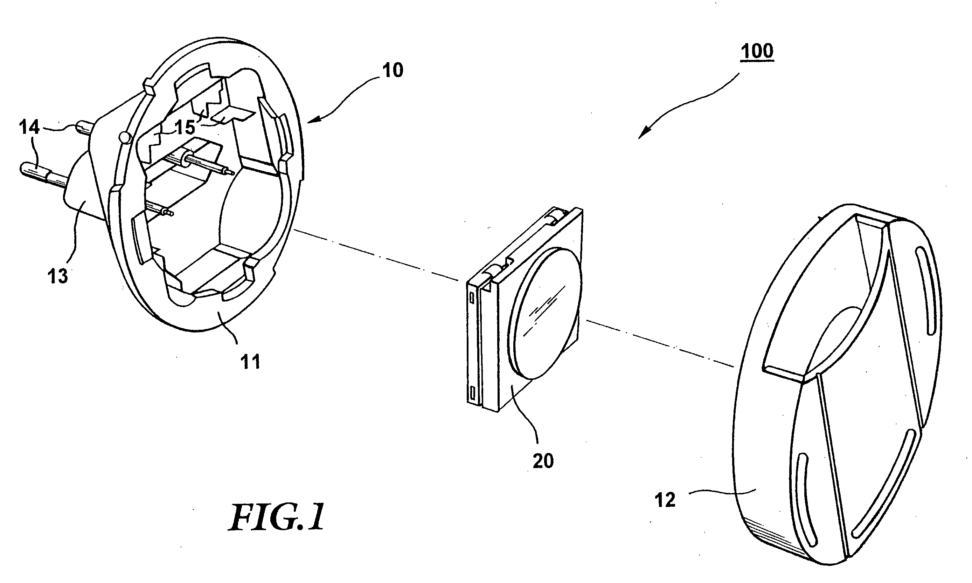

Bezugnehmend auf Fig. 1 besteht die elektrische Vorrichtung 100 aus einem zweiteiligen Gehäuse 10 und einer darin gelagerten elektrischen Schaltung 20. Das Gehäuse umfaßt ein Anschlußteil 11 und einen daran befestigbaren Deckel 12.

Das Anschlußteil 11 besitzt an seiner rückwärtigen Außenfläche einen vorstehenden Steckersockel 13, von dem zwei Anschlußstekkerstifte 14 vorstehen. Der Steckersockel und die Anschlußstekkerstifte sind zum Anschluß an eine genormte Wand oder Tischsteckdose geeignet geformt. Auf der Gehäuseinnenseite des Anschlußteils sind Haltestege 15 zur Halterung der elektrischen Schaltung 20 ausgebildet.

Der Deckel 12 ist mittels Rastvorsprüngen an dem Anschlußteil befestigbar und weist an seinem Umfangsrand eine Öffnung zum Einführen einer nicht gezeigten Wanne aus Kunststoff oder Metall, z.B. Aluminium in eine im Deckel ausgebildete Tasche auf, in der sich der zu verdunstende Wirkstoff befindet. Im Deckel befinden sich auch Belüftungsöffnungen zur Luftzirkulation durch das Innere des Gehäuses.

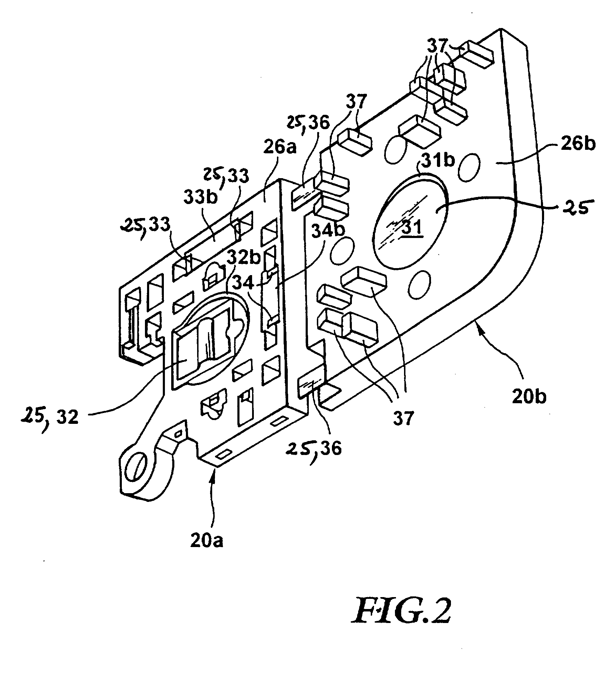

Im folgenden wird Bezug genommen auf Fig. 2, die im Detail die elektrische Schaltung 20 vor der Montage zeigt.

Die Schaltung besteht aus zwei Schaltungsteilen 20a, 20b, die durch Umspritzen der Unter- und Oberseite eines einstückigen Stanzblechteils 25 entstehen. Das Stanzblechteil weist bereits alle elektrischen Strompfade, Anschlußkontakte und Elektroden auf. Die Umspritzung ist ein ggf. mehrschichtig aufgetragenes Kunststoffisolationsmaterial, das die Steifigkeit des Stanzblechteils erhöht. Die Umspritzung ist so aufgebracht, daß zwei voneinander getrennte Kunststoffspritzgußteile 26a, 26b entstehen, die über zwei freie Stege 36 des einstückigen Stanzblechteils zusammengehalten sind.

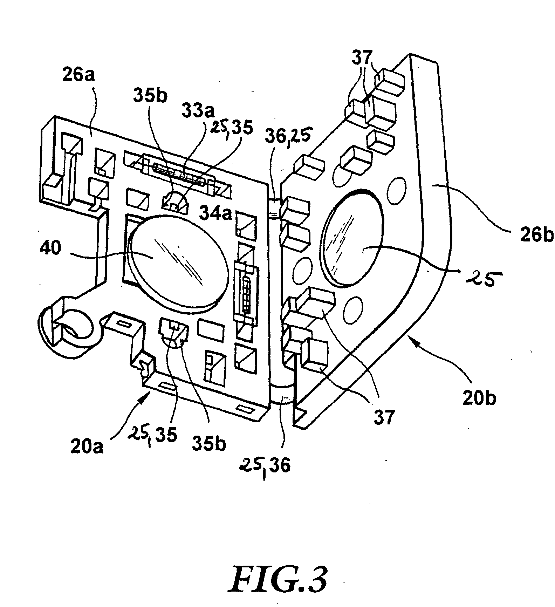

Das Stanzblechteil umfaßt eine erste Elektrode 31 und eine zweite Elektrode 32 mit einer federnden freigeschnittenen Zunge für ein PTC-Heizelement 40 (Fig. 3). Weiterhin umfaßt das Stanzblechteil 25 Anschlußkontakte 33, 34 für elektrische Widerstände 33a und für eine Kontrolleuchte 34a. Schließlich sind beidseitig der Elektrode 32 Anschlußfahnen 35 angeordnet, welche im zusammengebauten Zustand der Vorrichtung die Anschlußsteckerstifte 14 kontaktieren, um die Schaltung mit elektrischer Spannung versorgen zu können.

Die obengenannten Anschlüsse und Elektroden befinden sich jeweils in zugehörigen Ausnehmungen 31b bis 35b, die in das Kunststoffspritzgußteil 26a eingeformt sind und eine Aufnahme für die elektrischen Bauelemente der fertig montierten Schaltung bilden.

Den Ausnehmungen des einen Spritzgußteils 26a sind im Querschnitt rechteckige oder quadratischen Haltevorsprünge 37 auf dem anderen Spritzgußteil 26b zugeordnet.

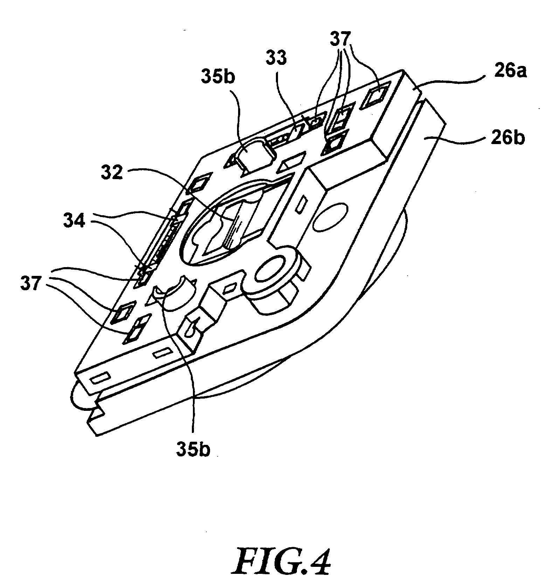

Die Figuren 3 und 4 zeigen die Schritte zur Herstellung einer vollständig bestückten elektrischen Schaltung 20. Man erkennt, daß die Stege 36 eine Art mechanisches Scharnier bilden, wobei das eine Kunststoffspritzgußteil 26a nach dem Einlegen der elektrischen Bauelemente auf das andere Kunststoffspritzgußteil 26b geklappt wird. Die Stege 36 werden beim Zusammenklappen um 180° gebogen. Im zusammengeklappten Zustand sind die Ausnehmungen 31b bis 35b mit den korrespondierenden Haltevorsprüngen 37 zur Verbindung der beiden Kunststoffspritzgußteile in Eingriff. Gegebenenfalls können durch das Vorsehen von Rastnasen an den Haltevorsprüngen 37 die beiden Kunststoffspritzgußteile fest miteinander verrastet werden.

Wie aus der Fig. 4 ersichtlich, biegen beim Zusammenklappen diejenigen Haltevorsprünge 37, denen Ausnehmungen 31b bis 35b zugeordnet sind, in denen ein elektrisches Bauteil aufgenommen ist, die waagerecht liegenden Anschlußdrähte der Bauteile 33a, 34a um 90°. Die Anschlußkontakte 33, 34 des Stanzblechs wirken dabei mit den Vorsprüngen 37 so zusammen, daß die Anschlußdrähte der elektrischen Bauteile klemmend zwischen ihnen gehalten sind. Dadurch wird eine sichere elektrische Verbindung zwischen den Anschlußkontakten und den Anschlußdrähten der elektrischen Bauteile hergestellt.

Die fertig zusammengebaute Schaltung 20 wird in das Anschlußteil 11 eingesetzt, wobei die Anschlußsteckerstifte 14 in den Ausnehmungen 35b, in denen sich die Anschlußfahnen 35 befinden, formschlüssig zu liegen kommen. Schließlich wird der Deckel 12 auf das Anschlußteil gesetzt und durch Drehen verrastet.

Die Vorrichtung kann zusätzlich mit einem nicht gezeigten Kippschalter versehen sein, um ein Unterbrechen der Spannungszufuhr auch ohne Herausziehen der Vorrichtung aus der Steckdose zu ermöglichen. In diesem Fall wird beispielsweise der Kippschalter an der elektrischen Schaltung befestigt und ist durch eine Öffnung im Gehäuse von außen bedienbar. Weiterhin kann die Vorrichtung einen Timer besitzen, der die Spannungszufuhr nach einer fest vorgegebenen oder einstellbaren Zeitdauer unterbricht und so eine sichere Abschaltung des Geräts gewährleistet.

## Patentansprüche

1. Elektrische Vorrichtung (100) zum Verdunsten von Wirkstoffen, Parfümen od. dgl., mit einem Gehäuse (10), in das ein den Wirkstoff enthaltender Behälter einsetzbar ist, mit einer im Gehäuse (10) gelagerten elektrischen Schaltung (20), bestehend aus zwei aufeinandergestapelten Schaltungsteilen (20a, 20b), zwischen denen ein PTC-Heizelement (40) angeordnet ist, wobei jedes Schaltungsteil ein Kunststoffspritzgußteil (26a, 26b) und ein darin eingebettetes Stanzblechteil (25) aufweist, **dadurch gekennzeichnet, daß** die beiden Schaltungsteile (20a, 20b) über ein einstückig mit den beiden Schaltungsteilen ausgebildetes Stanzblechteil (25) mechanisch und elektrisch miteinander gekoppelt sind wodurch die Schaltungsteile (20a, 20b) während der Montage der Vorrichtung wie ein Scharnier aufeinanderklappbar sind.

2. Elektrische Vorrichtung nach Anspruch 1, **dadurch gekennzeichnet, daß** in wenigstens ein Kunststoffspritzgußteil (26a) zur Aufnahme von diskreten elektrischen Bauelementen (33a, 34a) Ausnehmungen (33b, 34b) eingeformt sind.

3. Elektrische Vorrichtung nach Anspruch 2, **dadurch gekennzeichnet, daß** den Ausnehmungen (33b, 34b) des einen Kunststoffspritzgußteils (26a) korrespondierende hervorstehende Haltevorsprünge (37) auf dem anderen Spritzgußteil (26b) zugeordnet sind, die beim Aufeinanderklappen der Schaltungsteile die Anschlußdrähte der Bauelemente (33a, 34a) klemmend zwischen sich und dem Stanzblechteil (25) halten.

4. Elektrische Vorrichtung nach einem der Ansprüche 1 bis 3, **dadurch gekennzeichnet, daß** in wenigstens ein Spritzgußteil (26a) Ausnehmungen eingeformt sind, die mit entsprechenden Haltevorsprüngen (37) des anderen Spritzgußteils (26b) in rastenden Eingriff bringbar sind.

5. Elektrische Vorrichtung nach einem der Ansprüche 1 bis 4, **dadurch gekennzeichnet, daß** zur formschlüssigen Aufnahme von elektrischen Anschlußst:eckerstiften (14) in ein Kunststoffspritzgußteil (26a) entsprechende Aufnahmeöffnungen (35b) ausgebildet sind.

6. Elektrische Vorrichtung nach einem der Ansprüche 1 bis 5, **dadurch gekennzeichnet, daß** die Kopplung der Schaltungsteile (20a, 20b) über zwei zueinander parallele, freiliegende Stege (36) des Stanzblechteils (25) erfolgt.

## Claims

1. An electric device (100) for evaporating active substances, perfumes or the like, comprising a housing (10) adapted to receive a receptacle containing the active substance, and an electric circuit (20) supported in said housing (10) and consisting of two circuit components (20a, 20b) which are placed one on top of the other and between which a PCT heating element (40) is arranged, each of said circuit components comprising an injection molded part (26a, 26b) and a stamped sheet metal component (25) embedded therein, **characterized in that** said two circuit components (20a, 20b) are coupled to one another mechanically and electrically by means of a stamped sheet metal component (25) formed integrally with said two circuit components, whereby said circuit components (20a, 20b) can be folded during assembly of the device onto one another like a hinge.

2. An electric device according to claim 1, **characterized in that** said at least one injection molded part (26a) has formed therein openings (33b, 34b) for receiving discrete electric components (33a, 34a).

3. An electric device according to claim 2, **characterized in that** the openings (33b, 34b) provided in said one injection molded part (26a) have associated therewith complementary protruding retaining projections (37) which are provided on the other injection molded part (26b) and which clampingly hold the connecting wires of the components (33a, 34a) between them and the stamped sheet metal component (25) when the circuit components are folded onto one another.

4. An electric device according to one of claims 1 to 3, **characterized in that** at least one injection molded part (26a) has formed therein openings which are adapted to be brought into locking engagement with complementary retaining projections (37) of the other injection molded part (26b).

5. An electric device according to one of claims 1 to 4, **characterized in that** one injection molded part (26a) has formed therein corresponding receiving openings (35b) for positively receiving electric connecting pins (14).

6. An electric device according to one of claims 1 to 5, **characterized in that** the circuit components (20a, 20b) are coupled to one another via two parallel, exposed webs (36) of the stamped sheet metal component (25).

## Revendications

1. Dispositif électrique (100) pour la vaporisation de substances actives, de parfums ou analogues, avec un boîtier (10) dans lequel un récipient contenant la substance active est susceptible d'être inséré, avec un circuit électrique (20) monté dans le boîtier (10), constitué de deux parties de circuit (20a, 20b) empilées l'une sur l'autre, entre lesquelles est disposé un élément chauffant (40) à caractéristique de température positive (PTC), chaque partie de circuit présentant une partie moulée par injection en matière synthétique (26a, 26b) et une partie en tôle d'estampage (25) y étant noyée, **caractérisé en ce que** les deux parties de circuit (20a, 20b) sont accouplées ensemble mécaniquement et électriquement, par l'intermédiaire d'une pièce en tôle d'estampage (25), réalisée d'une seule pièce avec les deux parties de circuit, les parties de circuit (20a, 20b) étant susceptibles d'être rabattues l'une sur l'autre pendant le montage du dispositif, comme pour une charnière.

2. Dispositif électrique selon la revendication 1, **caractérisé en ce que** des logements (33b, 34b) sont creusés en au moins une pièce moulée par injection en matière synthétique(26a) pour recevoir des éléments de construction (33a, 34a) électriques discrets.

3. Dispositif électrique selon la revendication 2, **caractérisé en ce que**, aux évidements (33b, 34b) de la première pièce moulée par injection en matière synthétique (26a), sont associées, sur l'autre pièce moulée d'injection (26b), des tétons de maintien (37) faisant saillie, correspondants, qui, lors du rabattement l'une sur l'autre des parties de charnière, maintiennent les fils de raccordement des éléments de construction (33a, 34a) enserrés, entre elles et la pièce en tôle estampée (25).

4. Dispositif électrique selon l'une des revendications 1 à 3, **caractérisé en ce qu'**en au moins une pièce moulée par injection (26a) sont creusés des évidements, susceptibles d'être mis en prise par encliquetage avec des tétons de maintien (37) appartenant à l'autre pièce moulée par injection (26b).

5. Dispositif électrique selon l'une des revendications 1 à 4, **caractérisé en ce que** des ouvertures de logement (35b) correspondantes sont réalisées dans une pièce moulée par injection en matière synthétique (26a), en vue de recevoir, par une liaison avec ajustement de forme, des broches de connecteur de raccordement électriques (14).

6. Dispositif électrique selon l'une des revendications 1 à 5, **caractérisé en ce que** le couplage des parties de circuit (20a, 20b) s'effectue par l'intermédiaire de deux nervures (36), dégagées, parallèles entre elles, de la pièce en tôle estampée (25).
